(19) Europäisches Patentamt
European Patent Office
Office européen des brevets

(11) Publication number : **0 460 647 B1**

(12) # EUROPEAN PATENT SPECIFICATION

(45) Date of publication of patent specification : **02.03.94 Bulletin 94/09**

(51) Int. Cl.$^5$ : **C08F 2/50,** G03F 7/031, C07D 273/01

(21) Application number : **91109201.3**

(22) Date of filing : **05.06.91**

(54) **Oxaziridine photoinitiators for free-radical polymerizations.**

(30) Priority : **07.06.90 IT 2057990**

(43) Date of publication of application : **11.12.91 Bulletin 91/50**

(45) Publication of the grant of the patent : **02.03.94 Bulletin 94/09**

(84) Designated Contracting States : **AT BE CH DE DK ES FR GB LI NL SE**

(56) References cited :
**CHEMISCHE BERICHTE. vol. 100, 1967, WEINHEIM DE pages 2593 - 2599; E. SCHMITZ ET AL.: 'oxaziridine,V Herstellung und Umlagerung von 2-Acyl-oxaziridinen '**

(73) Proprietor : **MINISTERO DELL' UNIVERSITA' E DELLA RICERCA SCIENTIFICA E TECNOLOGICA**
**76, Lungotevere Thaon di Revel**
**I-00196 Roma (IT)**

(72) Inventor : **Gila, Liliana, Dr.**
**4, Viale Fratelli Brignone**
**I-13039 Trino Vercellese Vercelli (IT)**
Inventor : **Garbassi, Fabio, Dr.**
**6, Via C. Porta**
**I-28100 Novara (IT)**
Inventor : **Morini, Giampiero, Dr.**
**215, Via Emilia**
**I-27058 Voghera, Pavia (IT)**
Inventor : **Citterio, Attilio, Dr.**
**5, Piazza Piola**
**I-20132 Milano (IT)**

(74) Representative : **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**D-80803 München (DE)**

**EP 0 460 647 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a class of organic compounds which can be used as photoinitiators for free-radical polyme rization reactions.

In particular, the present invention relates to the use of compounds belonging to the class of oxaziridine derivatives as photoinitiators in free-radical polymerization reactions.

As is known, photoinitiators are sources of free radicals which can be activated photochemically and are used as initiators in the polymerization of, e.g., polymerizable, ethylenically-unsaturated substrates. The photoinitiated free-radical polymerizations have reached a great importance at the industrial level: photopolymerizable compositions are used, e.g., in the production of printing inks, paints, coatings, photoresists, adhesives.

In general, the products available as photoinitiators are compounds belonging to the following classes: aryl-ketones, peresters, benzoin ethers, acetophenones, and highly-halogenated aromatic hydrocarbons. The use of the latter compounds has recently been prohibited in view to their very high toxicity.

In order to be suitable at the industrial level, a photoinitiator system should be thermally stable and at the same time labile when irradiated with visible and/or U.V. light. Furthermore, to be suitable for a wider range of applications, the system should be active also when it is irradiated with different wavelengths.

Therefore, an object of the present invention is to provide a class of compounds which are both thermally stable and effectively photo-decomposable, also at different wavelengths, which renders them particularly suitable for use as photoinitiators in polymerization reactions of (ethylenically) unsaturated polymerizable substrates (e.g. monomers).

Another object is to provide a new class of photoinitiator compounds, the photodecomposition whereof can be controlled and regulated by suitably selecting the chromophoric portion present therein.

A further object is to provide compositions comprising at least one ethylenically unsaturated, polymerizable substrate and at least one of the photoinitiators of the present invention and the corresponding polymerization process by exposure to visible light or U.V radiation.

Still another object is to provide the polymers obtainable from the monomers polymerized by means of the photoinitiators of the present invention.

A class of novel benzoyl-benzoyl-oxaziridine compounds constitutes a further object of the present invention.

These and still other objects, which will become apparent to those skilled in the art from the following disclosure, are achieved, according to the present invention, by the provision of oxaziridine free-radical photoinitiators of general formula (I):

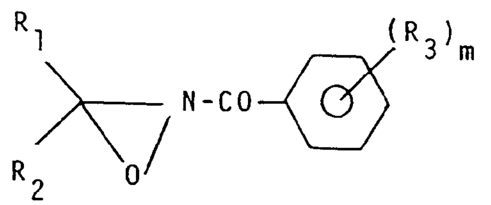

(I)

wherein:

$R_1$ and $R_2$, the same or different from each other, are selected from alkyl, aryl, alkyl-aryl and aryl-alkyl groups or, taken together, form a cycloalkyl group with the carbon atom to which they are bound;

$R_3$ is selected from alkyl, alkoxy, $NO_2$, halogen and R'CO, R' representing an alkyl, cycloalkyl, aryl, alkyl-aryl, arylalkyl or heterocyclic group, R' being optionally substituted with at least one group selected from $C_1$-$C_5$ alkoxy, $NO_2$ and halogen; and m is 0 or an integer of from 1 to 3;

Preferably, $R_1$ and $R_2$ represent a straight or branched alkyl group containing from 1 to 10, particularly 1 to 6, carbon atoms, an aryl group containing from 6 to 14 carbon atoms, an aryl-alkyl or an alkyl-aryl group containing from 7 to 18 carbon atoms or $R_1$ and $R_2$ are taken together to form a cycloalkyl ring containing from 3 to 10 (particularly 4 to 8) carbon atoms together with the carbon atom to which they are bound.

$R_3$ preferably represents a $C_1$-$C_5$ alkyl or $C_1$-$C_5$ alkoxy group, (e.g. methyl, ethyl and (iso)propyl and the corresponding alkoxy groups) $NO_2$, halogen (e.g. F, Cl, Br and I, particularly F and Cl) or R'CO wherein R' represents a linear or branched alkyl group containing from 1 to 20, particularly 1 to 10 carbon atoms, a cycloalkyl group containing from 3 to 12, particularly 4 to 8 carbon atoms, an aryl group containing from 6 to 14 carbon atoms, an alkyl-aryl or an aryl-alkyl group each containing from 7 to 20 carbon atoms or a heterocyclic group (preferably having 4 to 6 carbon atoms and at least one (e.g. 1 or 2) heteroatoms independently selected from O, N and S); said radicals may also be substituted with one or more groups. Substituents for R' are $C_1$-$C_5$ alkoxy,

$NO_2$ and halogen, said substituents being the same or different from each other when more than one is present.

Particularly preferred groups $R_1$ and $R_2$ are methyl, ethyl, propyl, isopropyl, and butyl; phenyl, anthracyl; benzyl, phenethyl, and phenylpropyl; tolyl, xylyl; (if taken together) ethylene, propylene, butylene, and pentylene.

Preferably, the linear or branched groups R' contain from 1 to 10 carbon atoms, such as methyl, ethyl, propyl, butyl, pentyl, and hexyl.

Examples of R' cycloalkyl groups are: cyclopropyl, cyclobutyl, cyclopentyl, cyclohexyl, cycloheptyl, and cyclooctyl.

Examples of aryl groups R' are phenyl, phenanthryl and anthracyl.

Examples of aralkyl groups R' are benzyl and naphthylethyl; and exemplary R' alkylaryl groups comprise tolyl, xylyl and cumyl.

Examples of heterocyclic groups R' are 5- or 6-membered rings containing carbon atoms and one or more of O, N, and/or S, such as morpholyl, piperidyl, thienyl and furanyl.

The preferred R'CO-group is the benzoyl group, which may also be substituted, such as 2,4,6-trimethyl- (or -trimethoxy-)benzoyl.

Preferably, said R'CO group is in the para-position with respect to the CO-group.

The latter class of benzoyl-benzoyl-oxaziridine derivatives is per se novel and constitutes a further object of the present invention.

Particularly satisfactory results may be obtained when using the following oxaziridines: 2-benzoyl-3-methyl-3-ethyl-oxaziridine; 2-benzoyl-3,3-pentamethylene-oxaziridine; 2-[(4-benzoyl)-benzoyl]-3-methyl-3-ethyl-oxaziridine; 2-[(4-benzoyl)-benzoyl]-3,3-pentamethylene-oxaziridine; 2-[(4-methyl)-benzoyl]-3-methyl-3-ethyl-oxaziridine; 2-[(4-nitro)-benzoyl]-3-methyl-3-propyl-oxaziridine; 2-benzoyl-3-methyl-3-isopropyloxaziridine;2-[(4-chloro)-benzoyl]-3-methyl-3-ethyl-oxaziridine and mixtures thereof.

The oxaziridine photoinitiator compounds of the present invention are per se known compounds, or can be synthesized according to methods known from literature.

For example, they can be prepared by acylating the corresponding N-H oxaziridines with an acyl chloride (Chem. Ber. 100, p. 2593-2599, 1967).

As already mentioned, the present invention also provides novel benzoyl-benzoyloxaziridine compounds of general formula (II):

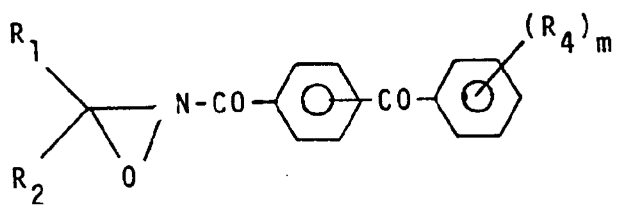

(II)

wherein:

$R_1$, $R_2$ and m have the meanings given above and $R_4$ represents a $C_1$-$C_5$ alkyl or $C_1$-$C_5$ alkoxy group, $NO_2$ or halogen (e.g. F or Cl).

These compounds may be obtained by reacting a suitable N-H oxaziridine in an ether solution with a suitably substituted benzoyl-benzoyl chloride, also dissolved in ether, in the presence of a weak base (e.g. $Na_2CO_3$) and at relatively low temperatures (e.g. 10 to 12°C).

The N-H oxaziridine may be obtained by amination of a suitable ketone with N-hydroxylamino-O-sulfonic acid in a basic aqueous medium, in the presence of a strong base and at a temperature which is preferably lower than 15°C. The benzoyl-benzoyl chloride may be obtained by chlorination, under reflux, of the corresponding acid with thionyl chloride.

The compounds of formula (I) and (II) described herein are particularly useful as photoinitiators for the polymerization of ethylenically unsaturated, polymerizable substrates, which may comprise ethylenically unsaturated monomers and/or prepolymers.

The oxaziridines (I) and (II) are usually employed as photoinitiators in molar ratios of from 1:100 to 1:2,000, and preferably of from 1:200 to 1:1,000 with respect to the polymerizable monomer or prepolymer. Mixtures of the free-radical photoinitiators (I) and (II) can also be used.

The polymerization can be carried out by exposing the compositions to be polymerized (i.e. essentially the monomer(s) and the photoinitiator), to visible or ultra-violet light, at a suitable wavelength. The wavelength should be selected in such a way that it can be absorbed by the chromophoric portion of the photoinitiator compound (I).

In this regard, it is to be pointed out that the photoinintiators of the present invention can be adapted, by properly selecting the substituents, to the particular requirements of absorption of radiation energy (relative to the available light sources), or to the type of substrate.

According to methods known in the art, usually a source of light is selected which emits radiation within the range of visible to U.V. light. Therefore, the chromophoric group is selected in such a way that it will absorb light having a wavelength ranging from 200 to 700 nm.

Examples of photopolymerizable materials suitable for the present invention comprise monomers or prepolymers containing one or more ethylenic unsaturation(s), such as the esters of unsaturated monocarboxylic or dicarboxylic acids, e.g., the esters of acrylic, methacrylic, sorbic, fumaric, maleic, itaconic acids with mono- or polyhydroxy aliphatic, cycloaliphatic and aromatic alcohols containing from 3 to 20 carbon atoms; the amides, N-alkylamides, N-alkoxyamides of acrylic and methacrylic acids; the vinyl esters of mono- and dicarboxylic acids containing from 2 to 20 carbon atoms; the vinyl ethers of monohydroxy or dihydroxy alcohols containing from 3 to 20 carbon atoms; the allyl esters of mono- or dicarboxylic acids; the allyl ethers of mono- or dihydroxy alcohols; vinyl- and vinylidene halo-derivatives; vinylic and styrenic aromatic compounds ; unsaturated polyurethanes; unsaturated polyesters.

The initiator agents (I) and (II) of the present invention are preferably used in the polymerization of methyl methacrylate, methyl acrylate, 2-ethylhexyl acrylate, hydroxyethyl acrylate and methacrylate, ethylene glycol diacrylate, neopentyl glycol diacrylate and dimethacrylate, N,N'-dimethylacrylamide, N-methoxymethacrylamide, N-butoxymethylmethacrylamide; vinyl acetate, vinyl propionate; isobutyl-vinyl-ether, hexyl-vinylether, ethylene glycol divinyl ether, hexanediol divinyl-ether; diallyl maleate, diallyl fumarate; styrene, divinyl-benzene, methylstyrene; and mixtures thereof.

The polymerization usually requires an exposure to light of from 30 seconds to 20 hours, said time varying, e.g., as a function of the wavelength, the intensity of the radiation used, the sensitivity of the chromophoric group present and the unsaturated substrate, when operating at temperatures within the range of from 10°C to 50°C, and preferably 20°C to 30°C.

The polymerization reactions photoinitiated by means of the photoinitiators of the present invention may be carried out according to methods known from the literature [J. Polym. Sci.: Polym. Chem. 21, 3129-3144 (1983)].

The following examples are given for merely illustrative but not limitative purposes.

## EXAMPLE 1

### Synthesis of 2-benzoyl-3-methyl-3-ethyloxaziridine

7.2 g (0.1 mole) of methylethylketone, dissolved in 50 ml of methylene chloride, were charged into a three-neck flask equipped with mechanical stirrer and thermometer and cooled in an ice bath. 50 ml of 2N NaOH and a solution of 12 g (0.1 mole) of N-hydroxylamino-O-sulphonic acid (at 97%) in 100 ml of water and 50 ml of 2N NaOH and, rapidly thereafter, 14 g (0.1 mole) of benzoyl chloride, were added, under vigorous stirring, keeping the temperature lower than 10°C for 10 minutes. The organic phase was then separated from the aqueous phase, washed with water and dried over anhydrous sodium sulphate. The crude reaction product, obtained by evaporation of the solvent, was purified by flash chromatography using hexane/ethylacetate (8:2) as eluent. 2 g of pure product were obtained (purity determined by iodometry: 99.5%).

According to the same synthesis scheme the following compounds were prepared: 2-benzoyl-3,3-pentamethylene-oxaziridine; 2-[(4-methyl)-benzoyl]-3-methyl-3-ethyl-oxaziridine; 2-[(4-nitro)-benzoyl]-3-methyl-3-propyl-oxaziridine; 2-benzoyl-3-methyl-3-isopropyl-oxaziridine; 2-[(4-chloro)-benzoyl]-3-methyl-3-ethyl-oxaziridine.

## EXAMPLE 2

### Synthesis of 2-[4-benzoyl)-benzoyl]-3,3-pentamethylene-oxaziridine

(a) Synthesis of 2-(4-benzoyl)-benzoyl chloride

12.7 ml (0.175 mol) of thionyl chloride and a few drops of pyridine were added to 8 g (0.035 mol) of 4-benzoyl-benzoic acid. The reaction mixture was heated up to the reflux temperature (about 80°C) and was kept at said temperature until the reaction was complete (i.e., until the evolution of hydrogen chloride had ceased).

8 g of acyl chloride were obtained. Before use, the crude reaction product was purified by crystallization from 1:1 ether/pentane.

(b) Synthesis of 3,3-pentamethylene-oxaziridine

9.8 g (0.1 mole) of cyclohexanone, dissolved in 170 ml of ether, were charged into a three-neck flask equipped with mechanical stirrer and thermometer. 50 ml of NaOH (2N) and rapidly thereafter 12 g (0.1 mole) of N-hydroxylamino-O-sulphonic acid (at 97%), dissolved in 100 ml of water, and 50 ml of NaOH (2N) were added to the solution, cooled to 4-5°C and kept under vigorous stirring, maintaining the temperature below 10°C. After about 10 minutes, when the temperature started to decrease, the organic phase was separated from the aqueous phase, washed with water and dried over anhydrous sodium sulphate. The product was stored in solution at 4°C. The 3,3-pentamethylene-oxaziridine titre was determined by iodometric titration:
30 ml of glacial acetic acid, 50 ml of water and 14 ml of an aqueous solution of KI at 10% were added to 5 ml of the oxaziridine solution. Then the solution was titrated with a solution of sodium thiosulphate (0.1 N).
5 g of product were obtained.

(c) Synthesis of 2-[(4-benzoyl)-benzoyl]-3,3-pentamethylene-oxaziridine

30 mmoles of 3,3-pentamethylene oxaziridine in diethylether (about 100 ml) were charged into a three-neck flask equipped with mechanical stirrer and thermometer and cooled at 4 to 5°C.

7.5 ml of a 2M $Na_2CO_3$ solution and 20 ml of water and, rapidly thereafter, 7.3 g (30 mmoles) of 4-benzoyl-benzoyl chloride in 200 ml of ether were added under vigorous stirring.

The reaction mixture was kept at 5 to 10°C for 10 minutes. The organic phase was then separated from the aqueous phase, washed with water and dried over anhydrous sodium sulphate.

The crude reaction product, obtained by evaporation of the solvent, was purified by flash chromatography using hexane/ ethylacetate (8:2) as eluent.

2.8 g of pure product were obtained (iodometric purity: 98%) as white solid having a melting point of 110-111°C.

IR (KBr): ketonic $\nu_{CO}$ = 1650 $cm^{-1}$; oxaziridinic $\nu_{CO}$ = 1700 $cm^{-1}$.

EXAMPLE 3

Synthesis of 2-[(4-benzoyl)-benzoyl]-3-methyl-3-ethyl-oxaziridine

The synthesis process of example 2 was used to prepare 2-[(4-benzoyl)-benzoyl]-3-methyl-3-ethyl-oxaziridine.

30 mmoles of 3-methyl-3-ethyl-oxaziridine in methylene chloride, prepared according to the synthesis scheme of example 2b, were treated with 7.3 g (30 mmoles) of 4-benzoyl-benzoyl chloride in methylene chloride, prepared from 4-benzoyl-benzoic acid according to the synthesis scheme of example 2a, in the presence of an aqueous solution of $Na_2CO_3$.

6 g of pure product (iodometric purity: 98%) were obtained as white solid having a melting point of 90-91°C.

IR (KBr): ketonic $\nu_{CO}$ = 1650 $cm^{-1}$; oxaziridinic $\nu_{CO}$ = 1710 $cm^{-1}$.

EXAMPLES 4 TO 9

Photopolymerization of methyl methacrylate (MMA)

(a) Preparation of sample

Commercial methyl methacrylate (MMA) was washed 3 times with a 2% solution of NaOH (to remove the stabilizer), then 3 times with distilled water, dried over anhydrous $CaCl_2$ and vacuum-distilled before use.

8 ml of monomer, exactly measured, were poured into a Pyrex glass (or quartz) test tube equipped with a stopcock and then were degassed under vacuum by the "freeze/thaw" method. The freeze/thaw cycle was repeated 3 times.

Then an exactly weighed amount of photoinitiator, such as to obtain a solution with a known concentration (initiator/monomer molar ratio 1:900), was added to the frozen monomer in a nitrogen atmosphere. The solution was stored under nitrogen, and was kept protected from light until it was subjected to irradiation.

(b) Photopolymerization

The photopolymerizations were carried out in an annular photoreactor (APQ 40 Model, by Applied Photophysics Ltd.) by placing a set of samples (maximum 8 samples) into a suitable sample holder, revolving around the U.V. lamp and immersed into a water bath, and by irradiating the solutions for a predetermined time, at 20°C.

At the end of the irradiation, the test tubes were opened and their contents were poured into an excess of methanol (10:1 volume/volume).

The precipitated polymer was filtered off, washed with methanol, dried under vacuum and weighed.

The polymer was then characterized by GPC.

The photoinitiators used, the irradiation times and wavelengths (λ), the conversions and the average molecular weights obtained are listed in the following Table 1.

TABLE I

| EXAMPLE No. | PHOTOINITIATOR | IRRADIATION TIME (h) | IRRADIATION λ (nm) | CONVERSION (%) | Mn | Mw | Mw/Mn |
|---|---|---|---|---|---|---|---|
| 4 | 2-BENZOYL-3,3-PENTAMETHYLENE OXAZIRIDINE | 0.5 | 366 | 2.1 | 31300 | 97400 | 3.1 |
| 5 | 2-BENZOYL-3-METHYL-3-ETHYL-OXAZIRIDINE | 0.5 | 366 | 1.9 | 38200 | 117600 | 3.1 |
| 6 | 2-[(4-BENZOYL)-BENZOYL]-3,3-PENTAMETHYLENE-OXAZIRIDINE | 0.5 | 366 | 2.3 | 25700 | 59300 | 2.3 |
| 7 | 2-[(4-BENZOYL)-BENZOYL]-3-METHYL-3-ETHYL-OXAZIRIDINE | 0.5 | 366 | 5.6 | 23000 | 68100 | 3.0 |
| 8 | 2-BENZOYL-3-METHYL-3-ETHYL-OXAZIRIDINE | 2 | 254 | 1 | 255870 | 1139800 | 4.4 |
| 9 | 2-[(4-BENZOYL)-BENZOYL]-3-METHYL-3-ETHYL-OXAZIRIDINE | 2 | 254 | 2.9 | 293300 | 1014700 | 3.5 |

## EXAMPLES 10 AND 11

### Styrene photopolymerization

Commercial styrene was purified before use, as described in examples 4 to 9.

The preparation of the sample, the photopolymerization equipment and the treatments for polymer precipitation and isolation also were identical to those described in examples 4 to 9.

The photoinitiators used, the irradiation times and wavelengths ($\lambda$), the conversions and the average molecular weights obtained are listed in the following Table 2.

TABLE 2

| EXAMPLE No. | PHOTOINITIATOR | IRRADIATION TIME (h) | IRRADIATION λ (nm) | CONVERSION (%) | Mn | Mw | Mw/Mn |
|---|---|---|---|---|---|---|---|
| 10 | 2-BENZOYL-3-METHYL-3-ETHYL-OXAZIRIDINE - | 4 | 366 | 1.2 | 1100 | 29860 | 2.7 |
| 11 | 2-[(4-BENZOYL)-BENZOYL]-3-METHYL-3-ETHYL-OXAZIRIDINE | 4 | 366 | 1.3 | 15100 | 70620 | 4.7 |

## Claims

### Claims for the following Contracting States : AT, BE, CH, DE, DK, FR, GB, LI, NL, SE

1. Use of oxaziridine compounds of general formula (I):

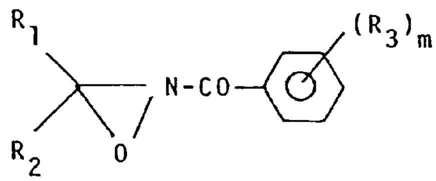

(I)

wherein:
$R_1$ and $R_2$, the same or different from each other, are selected from alkyl, aryl, alkyl-aryl and aryl-alkyl groups or, taken together, form a cycloalkyl group with the carbon atom to which they are bound;
$R_3$ is selected from alkyl, alkoxy, $NO_2$, halogen and R'CO, R' representing an alkyl, cycloalkyl, aryl, alkyl-aryl, arylalkyl or heterocyclic group, R' being optionally substituted with at least one group selected from $C_1$-$C_5$ alkoxy, $NO_2$ and halogen; and m is 0 or an integer of from 1 to 3; as free radical photoinitiators for photopolymerization reactions.

2. Use according to claim 1, wherein $R_1$ and $R_2$ are independently selected from linear or branched $C_1$-$C_{10}$ alkyl, $C_6$-$C_{14}$ aryl, $C_7$-$C_{18}$ aryl-alkyl or $C_7$-$C_{18}$ alkyl-aryl or $R_1$ and $R_2$, taken together, form a $C_3$-$C_{10}$ cycloalkyl group with the carbon atom to which they are bound; and/or $R_3$ represents $C_1$-$C_5$ alkyl or $C_1$-$C_5$ alkoxy and R' is selected from $C_1$-$C_{20}$ alkyl, $C_3$-$C_{12}$ cycloalkyl, $C_6$-$C_{14}$ aryl, $C_7$-$C_{20}$ alkyl-aryl, $C_7$-$C_{20}$ aryl-alkyl and heterocyclic groups containing 5 or 6 atoms and R' being optionally substituted with at least one group selected from $C_1$-$C_5$ alkoxy, $NO_2$ and halogen.

3. Use according to any one of the preceding claims, wherein $R_3$ is R'CO.

4. Use according to claim 1, wherein the oxaziridine compound is selected from 2-benzoyl-3,3-pentamethylene-oxaziridine; 2-benzoyl-3-methyl-3-methyl-oxaziridine; 2-[(4-benzoyl)-benzoyl]-3,3-pentamethylene-oxaziridine; 2-[4-benzoyl)-benzoyl]-3-methyl-3-ethyl-oxaziridine; 2-[(4-methyl)-benzoyl]-3-methyl-3-ethyl-oxaziridine; 2-[(4-nitro)-benzoyl]-3-methyl-3-propyl-oxaziridine; 2-benzoyl-3-methyl-3-isopropyl-oxaziridine; 2-[(4-chloro)-benzoyl]-3-methyl-3-ethyl-oxaziridine; and mixtures thereof.

5. Photopolymerizable composition comprising at least one ethylenically unsaturated, polymerizable substrate and at least one oxaziridine compound as defined in any one of claims 1 to 4.

6. Composition according to claim 5, wherein the molar ratio of oxaziridine compound to polymerizable substrate is from 1/100 to 1/2000, particularly from 1/200 to 1/1000.

7. Composition according to any one of claims 5 and 6, wherein the ethylenically unsaturated substrate comprises methyl methacrylate and/or styrene.

8. Process for polymerizing a photopolymerizable composition as defined in any one of claims 5 to 7, comprising exposing said composition to radiation having a wavelength of from 200 to 700 nm, preferably at a temperature of from 10 to 50°C, particularly of from 20 to 30°C, for 30 seconds to 20 hours.

9. Benzoyl-benzoyl-oxaziridines of general formula (II):

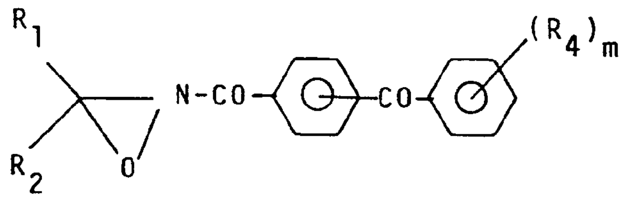

(II)

wherein:
$R_1$, $R_2$ and m have the meanings given in any one of claims 1 and 2 and $R_4$ represents a $C_1$-$C_5$ alkyl or $C_1$-$C_5$ alkoxy group, $NO_2$ or halogen.

**10.** Compounds according to claim 9, selected from 2-[(4-benzoyl)-benzoyl]-3-methyl-3-ethyl-oxaziridine or 2-[(4-benzoyl)-benzoyl]-3,3-pentamethylene-oxaziridine.

**11.** Polymers obtainable by photopolymerizing the compositions according to any one of claims 5 to 7.

**Claims for the following Contracting State : ES**

**1.** Use of oxaziridine compounds of general formula (I):

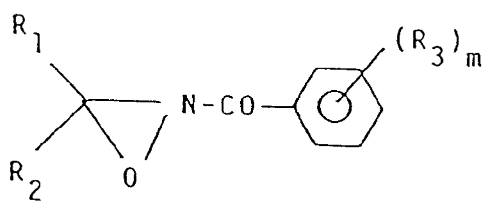

(I)

wherein:
$R_1$ and $R_2$, the same or different from each other, are selected from alkyl, aryl, alkyl-aryl and aryl-alkyl groups or, taken together, form a cycloalkyl group with the carbon atom to which they are bound;
$R_3$ is selected from alkyl, alkoxy, $NO_2$, halogen and R'CO, R' representing an alkyl, cycloalkyl, aryl, alkyl-aryl, arylalkyl or heterocyclic group, R' being optionally substituted with at least one group selected from $C_1$-$C_5$ alkoxy, $NO_2$ and halogen; and m is 0 or an integer of from 1 to 3;
as free radical photoinitiators for photopolymerization reactions.

**2.** Use according to claim 1, wherein $R_1$ and $R_2$ are independently selected from linear or branched $C_1$-$C_{10}$ alkyl, $C_6$-$C_{14}$ aryl, $C_7$-$C_{18}$ aryl-alkyl or $C_7$-$C_{18}$ alkyl-aryl or $R_1$ and $R_2$, taken together, form a $C_3$-$C_{10}$ cycloalkyl group with the carbon atom to which they are bound; and/or $R_3$ represents $C_1$-$C_5$ alkyl or $C_1$-$C_5$ alkoxy and R' is selected from $C_1$-$C_{20}$ alkyl, $C_3$-$C_{12}$ cycloalkyl, $C_6$-$C_{14}$ aryl, $C_7$-$C_{20}$ alkyl-aryl, $C_7$-$C_{20}$ aryl-alkyl and heterocyclic groups containing 5 or 6 atoms and R' being optionally substituted with at least one group selected from $C_1$-$C_5$ alkoxy, $NO_2$ and halogen.

**3.** Use according to any one of the preceding claims, wherein $R_3$ is R'CO.

**4.** Use according to claim 1, wherein the oxaziridine compound is selected from 2-benzoyl-3,3-pentamethylene-oxaziridine; 2-benzoyl-3-methyl-3-ethyl-oxaziridine; 2-[(4-benzoyl)-benzoyl]-3,3-pentamethylene-oxaziridine; 2-[4-benzoyl)-benzoyl]-3-methyl-3-ethyl-oxaziridine; 2-[(4-methyl)-benzoyl]-3-methyl-3-ethyl-oxaziridine; 2-[(4-nitro)-benzoyl]-3-methyl-3-propyl-oxaziridine; 2-benzoyl-3-methyl-3-isopropyl-oxaziridine; 2-[(4-chloro)-benzoyl]-3-methyl-3-ethyl-oxaziridine; and mixtures thereof.

**5.** Photopolymerizable composition comprising at least one ethylenically unsaturated, polymerizable substrate and at least one oxaziridine compound as defined in any one of claims 1 to 4.

**6.** Composition according to claim 5, wherein the molar ratio of oxaziridine compound to polymerizable substrate is from 1/100 to 1/2000, particularly from 1/200 to 1/1000.

**7.** Composition according to any one of claims 5 and 6, whe-rein the ethylenically unsaturated substrate comprises methyl methacrylate and/or styrene.

8. Process for polymerizing a photopolymerizable composition as defined in any one of claims 5 to 7, comprising exposing said composition to radiation having a wavelength of from 200 to 700 nm, preferably at a temperature of from 10 to 50°C, particularly of from 20 to 30°C, for 30 seconds to 20 hours.

9. Polymers obtainable by photopolymerizing the compositions according to any one of claims 5 to 7.

## Patentansprüche

### Patentansprüche für folgende Vertragsstaaten : AT, BE, CH, DE, DK, FR, GB, LI, NL, SE

1. Verwendung von Oxaziridin-Verbindungen der allgemeinen Formel (I):

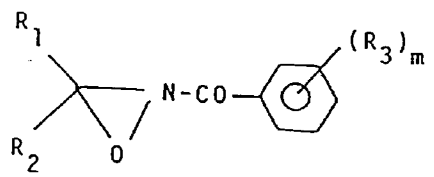

(I)

worin:
$R_1$ und $R_2$, die gleich oder verschieden sind, ausgewählt werden aus Alkyl-, Aryl-, Alkylaryl- und Arylalkylgruppen, oder die zusammen mit dem Kohlenstoffatom, an das sie gebunden sind, eine Cycloalkylgruppe bilden;
$R_3$ ausgewählt ist aus Alkyl, Alkoxy, $NO_2$, Halogen und R'CO, wobei R' eine Alkyl-, Cycloalkyl-, Aryl-, Alkylaryl-, Arylalkyl oder heterozyklische Gruppe bedeutet und gegebenenfalls mit wenigstens einer Gruppe, ausgewählt aus $C_1$-$C_5$-Alkoxy, $NO_2$ und Halogen, substituiert ist; m 0 oder eine ganze Zahl von 1 bis 3 ist;
als freiradikalische Photoinitiatoren für Photopolymerisationsreaktionen.

2. Verwendung nach Anspruch 1, worin $R_1$ und $R_2$ unabhängig voneinander ausgewählt werden aus linearem oder verzweigtem $C_1$-$C_{10}$-Alkyl, $C_6$-$C_{14}$-Aryl, $C_7$-$C_{18}$-Arylalkyl oder $C_7$-$C_{18}$-Alkylaryl, oder $R_1$ und $R_2$ zusammen mit dem Kohlenstoffatom, an das sie gebunden sind, eine $C_3$-$C_{10}$-Cycloalkylgruppe bilden; und/oder $R_3$ $C_1$-$C_5$-Alkyl oder $C_1$-$C_5$-Alkoxy bedeutet, und R' ausgewählt wird aus $C_1$-$C_{20}$,-Alkyl, $C_3$-$C_{12}$-Cycloalkyl, $C_{6-14}$-Aryl, $C_7$-$C_{20}$-Alkylaryl, $C_7$-$C_{20}$-Arylalkyl und heterozyklischen Gruppen mit 5 oder 6 Atomen, und R'gegebenenfalls substituiert sein kann mit wenigstens einer Gruppe, ausgewählt aus $C_1$-$C_5$-Alkoxy, $NO_2$ und Halogen.

3. Verwendung nach einem der vorhergehenden Ansprüche, worin $R_3$ R'CO ist.

4. Verwendung nach Anspruch 1, worin die Oxaziridin-Verbindung ausgewählt wird aus 2-Benzoyl-3,3-pentamethylen-oxaziridin-1 2-Benzoyl-3-methyl-3-ethyl-oxaziridin; 2-[(4-Benzoyl)-benzoyl]-3,3-pentamethylen-oxaziridin;2-[(4-Benzoyl)-benzoyl]-3-methyl-3-ethyl-oxaziridin; 2-[(4-Methyl)-benzoyl]-3-methyl-3-ethyl-oxaziridin; 2-[(4-Nitro)-benzoyl]-3-methyl-3-propyl-oxaziridin; 2-Benzoyl-3-methyl-3-isopropyl-oxaziridin; 2-[(4-Chlor)benzoyl]-3-methyl-3-ethyl-oxziridin und Mischungen von diesen.

5. Photopolymerisierbare Zusammensetzung, die wenigstens ein ethylenisch ungesättigtes, polymerisierbares Substrat und wenigstens eine Oxaziridin-Verbindung nach einem der Ansprüche 1 bis 4 umfaßt.

6. Zusammensetzung nach Anspruch 5, worin das Molverhältnis der Oxaziridin-Verbindung zu dem polymerisierbaren Substrat von 1/100 bis 1/2.000, insbesondere von 1/200 bis 1/1.000, beträgt.

7. Zusammensetzung nach einem der Ansprüche 5 und 6, worin das ethylenisch ungesättigte Substrat Methylmethacrylat und/oder Styrol umfaßt.

8. Verfahren zur Polymerisation einer Photopolymerisierbaren Zusammensetzung nach einem der Ansprüch 5 bis 7, welches das Bestrahlen der genannten Zusammensetzung mit einer Wellenlänge von 200 bis 700 nm, vorzugsweise bei einer Temperatur von 10 bis 50° C, insbesondere von 20 bis 30° C, für eine Dauer von 30 Sekunden bis 20 Stunden, umfaßt.

9. Benzoyl-benzoyl-oxaziridin der allgemeinen Formel (II)

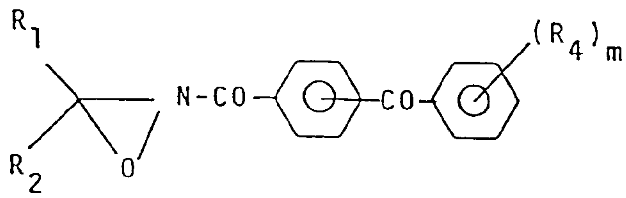

(II)

worin:
$R_1$ und $R_2$ die gleiche Bedeutung wie in einem der Ansprüche 1 und 2 haben und $R_4$ eine $C_1$-$C_5$-Alkyl- oder $C_1$-$C_5$-Alkoxygruppe, $NO_2$ oder Halogen bedeutet.

10. Verbindungen nach Anspruch 9, ausgewählt aus 2-[(4-Benzoyl)-benzoyl]-3-methyl-3-ethyl-oxaziridin oder 2-[(4-Benzoyl)-benzoyl]-3,3-pentamethylen-oxaziridin.

11. Polymere, erhältlich durch Photopolymerisation der Zusammensetzungen nach einem der Ansprüche 5 bis 7.

**Patentansprüche für folgenden Vertragsstaat : ES**

1. Verwendung von Oxaziridin-Verbindungen der allgemeinen Formel (I):

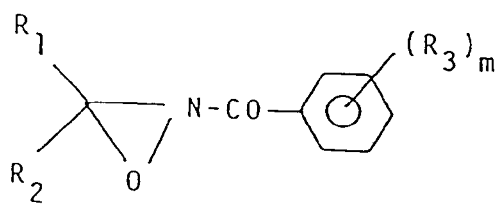

(I)

worin:
$R_1$ und $R_2$, die gleich oder verschieden sind, ausgewählt werden aus Alkyl-, Aryl-, Alkylaryl- und Arylalkylgruppen, oder die zusammen mit dem Kohlenstoffatom, an das sie gebunden sind, eine Cycloalkylgruppe bilden;
$R_3$ ausgewählt ist aus Alkyl, Alkoxy, $NO_2$, Halogen und R'CO, wobei R' eine Alkyl-, Cycloalkyl-, Aryl-, Alkylaryl-, Arylalkyl oder heterozyklische Gruppe bedeutet und gegebenenfalls mit wenigstens einer Gruppe, ausgewählt aus $C_1$-$C_5$-Alkoxy, $NO_2$ und Halogen, substituiert ist; m 0 oder eine ganze Zahl von 1 bis 3 ist;
als freiradikalische Photoinitiatoren für Photopolymerisationsreaktionen.

2. Verwendung nach Anspruch 1, worin $R_1$ und $R_2$ unabhängig voneinander ausgewählt werden aus linearem oder verzweigtem $C_1$-$C_{10}$-Alkyl, $C_6$-$C_{14}$-Aryl, $C_7$-$C_{18}$-Arylalkyl oder $C_7$-$C_{18}$-Alkylaryl, oder $R_1$ und $R_2$ zusammen mit dem Kohlenstoffatom, an das sie gebunden sind, eine $C_3$-$C_{10}$-Cycloalkylgruppe bilden; und/oder $R_3$ $C_1$-$C_5$-Alkyl oder $C_1$-$C_5$-Alkoxy bedeutet, und R' ausgewählt wird aus $C_1$-$C_{20}$-Alkyl, $C_3$-$C_{12}$-Cycloalkyl, $C_{6-14}$-Aryl, $C_7$-$C_{20}$-Alkylaryl, $C_7$-$C_{20}$-Arylalkyl und heterozyklischen Gruppen mit 5 oder 6 Atomen, und R' gegebenenfalls substituiert sein kann mit wenigstens einer Gruppe, ausgewählt aus $C_1$-$C_5$-Alkoxy, $NO_2$ und Halogen.

3. Verwendung nach einem der vorhergehenden Ansprüche, worin $R_3$ R'CO ist.

4. Verwendung nach Anspruch 1, worin die Oxaziridin-Verbindung ausgewählt wird aus 2-Benzoyl-3,3-pentamethylen-oxaziridin; 2-Benzoyl-3-methyl-3-ethyl-oxaziridin; 2-[(4-Benzoyl)-benzoyl]-3,3-pentamethylen-oxaziridin;2-[(4-Benzoyl)-benzoyl]-3-methyl-3-ethyl-oxaziridin; 2-[(4-Methyl)-benzoyl]-3-methyl-3-ethyl-oxaziridin; 2-[(4-Nitro)-benzoyl]-3-methyl-3-propyl-oxaziridin; 2-Benzoyl-3-methyl-3-isopropyl-oxaziridin; 2-[(4-Chlor)-benzoyl]-3-methyl-3-ethyl-oxziridin und Mischungen von diesen.

5. Photopolymerisierbare Zusammensetzung, die wenigstens ein ethylenisch ungesättigtes, polymerisierbares Substrat und wenigstens eine Oxaziridin-Verbindung nach einem der Ansprüche 1 bis 4 umfaßt.

6. Zusammensetzung nach Anspruch 5, worin das Molverhältnis der Oxaziridin-Verbindung zu dem polymerisierbaren Substrat von 1/100 bis 1/2.000, insbesondere von 1/200 bis 1/1.000, beträgt.

7. Zusammensetzung nach einem der Ansprüche 5 und 6, worin das ethylenisch ungesättigte Substrat Methylmethacrylat und/oder Styrol umfaßt.

8. Verfahren zur Polymerisation einer photopolymerisierbaren Zusammensetzung nach einem der Ansprüch 5 bis 7, welches das Bestrahlen der genannten Zusammensetzung mit einer Wellenlänge von 200 bis 700 nm, vorzugsweise bei einer Temperatur von 10 bis 50° C, insbesondere von 20 bis 30° C, für eine Dauer von 30 Sekunden bis 20 Stunden, umfaßt.

9. Polymere, erhältlich durch Photopolymerisation der Zusammensetzungen nach einem der Ansprüche 5 bis 7.

## Revendications

### Revendications pour les Etats contractants suivants : AT, BE, CH, DE, DK, FR, GB, LI, NL, SE

1. Utilisation à titre de photoinitiateurs à radicaux libres pour des réactions de photopolymérisation des composés de formule générale (I):

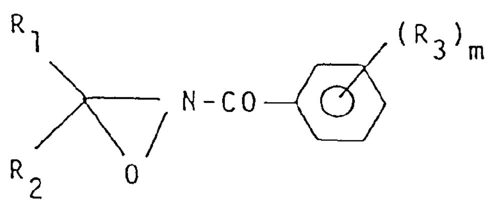

(I)

dans laquelle:

$R_1$ et $R_2$ identiques ou différents l'un de l'autre, sont choisis parmi les radicaux alkyle, aryle, alkylaryle et arylalkyle ou, pris ensemble, forment un cycle cycloalkyle avec l'atome de carbone auquel ils sont liés;

$R_3$ est choisi parmi les radicaux alkyle, alcoxy, un groupe $NO_2$, halogène et R'CO, R' représentant un groupe alkyle, cycloalkyle, aryle, alkylaryle, arylalkyle ou hétérocyclique et R' étant éventuellement substitué par au moins un groupe choisi dans le groupe comprenant les radicaux alcoxy en $C_1$ à $C_5$, un groupe $NO_2$ et halogène;

m étant égal à 0 ou à un nombre entier de 1 à 3.

2. Utilisation selon la revendication 1, dans laquelle $R_1$ et $R_2$ sont indépendamment choisis parmi les radicaux liéaires ou ramifiés alkyle en $C_1$ à $C_{10}$, aryle en $C_6$ à $C_{14}$, arylalkyle en $C_7$ à $C_{18}$ ou alkylaryle en $C_7$ à $C_{18}$, ou encore $R_1$ et $R_2$ pris ensemble forment un cycle cycloalkyle en $C_3$ à $C_{10}$, avec l'atome de carbone auquel ils sont liés, et/ou $R_3$ consiste en un radical alkyle en $C_1$ à $C_5$ ou alcoxy en $C_1$ à $C_5$, et R' est choisi dans le groupe comprenant les radicaux alkyle en $C_1$ à $C_{20}$, cycloalkyle en $C_3$ à $C_{12}$, aryle en $C_6$ à $C_{14}$, alkylaryle en $C_7$ à $C_{20}$, arylalkyle en $C_7$ à $C_{20}$ et les radicaux hétérocycliques contenant 5 ou 6 atomes et R' pouvant être éventuellement substitué par au moins un groupe choisi parmi les radicaux alcoxy en $C_1$ à $C_5$, un groupe $NO_2$ et halogène.

3. Utilisation selon l'une quelconque des revendications précédentes, dans laquelle $R_3$ est R'CO.

4. Utilisation selon la revendication 1, dans laquelle les composés oxaziridine consistent en:

- 2-benzoyl-3,3-pentaméthylène-oxaziridine;
- 2-benzoyl-3-méthyl-3-éthyl-oxaziridine;
- 2-[(4-benzoyl)-benzoyl]-3,3-pentaméthylène-oxaziridine;
- 2-[(4-benzoyl)-benzoyl]-3-méthyl-3-éthyl-oxaziridine;
- 2-[(4-méthyl)-benzoyl]-3-méthyl-3-éthyl-oxaziridine;
- 2-[(4-nitro)-benzoyl]-3-méthyl-3-propyl-oxaziridine;
- 2-benzoyl-3-méthyl-3-isopropyl-oxaziridine;
- 2-[(4-chloro)-benzoyl]-3-méthyl-3-éthyl-oxaziridine;

ainsi que leurs mélanges.

5. Composition photopolymérisable comprenant au moins un substrat polymérisable à saturation éthylénique et au moins un composé oxaziridine tel que défini dans l'une quelconque des revendications 1 à 4.

6. Composition selon la revendication 5, dans laquelle le rapport molaire composé oxaziridine/substrat polymérisable est compris entre 1/100 et 1/2000, notamment de 1/200 à 1/1000 .

7. Composition selon l'une quelconque des revendications 5 et 6, caractérisée en ce que le substrat à insatura-tion éthylénique comprend du méthacrylate de méthyle et/ou du styrène.

8. Procédé de polymérisation d'une composition photo-polymérisable telle que définie dans l'une quelconque des revendications 5 à 7, comprenant l'exposition de ladite composition à des radiations dont la longueur d'onde est comprise entre 200 à 700 nm, de préférence à une température comprise entre 10 et 50°C, tout particulièrement comprise entre 20 et 30°C, pendant 30 secondes à 20 heures.

9. Benzoyl-benzoyl-oxaziridines de formule générale (II):

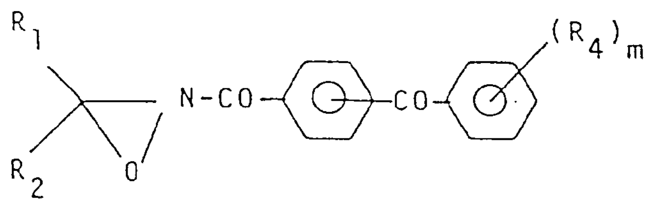

(II)

dans laquelle:

$R_1$, $R_2$ et m ont les significations données dans l'une quelconque des revendications 1 et 2; et

$R_4$ représente un radical alkyle en $C_1$ à $C_5$ ou alcoxy en $C_1$ à $C_5$, un groupe un groupe $NO_2$ ou un halogène.

10. Composés selon la revendication 9, consistant en 2-[(4-benzoyl)-benzoyl]-3-méthyl-3-éthyl-oxaziridine; ou 2-[(4-benzoyl)-benzoyl]-3,3-pentaméthylène-oxaziridine.

11. Polymères pouvant être obtenus par photopolymérisa-tion des compositions selon l'une quelconque des revendications 5 à 7.

**Revendications pour l'Etat contractant suivant : ES**

1. Utilisation des dérivés d'oxaziridine de formule générale (I):

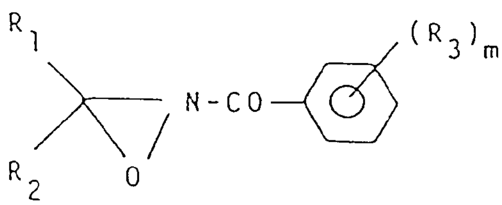

(I)

dans laquelle:

$R_1$ et $R_2$ identiques ou différents l'un de l'autre, sont choisis parmi les radicaux alkyle, aryle, alkylaryle et arylalkyle ou, pris ensemble, forment un cycle cycloalkyle avec l'atome de carbone auquel ils sont liés;

$R_3$ est choisi parmi les radicaux alkyle, alcoxy, un groupe $NO_2$, halogène et R'CO, R' représentant un groupe alkyle, cycloalkyle, aryle, alkylaryle, arylalkyle ou hétérocyclique et R' étant éventuellement substitué par au moins un groupe choisi dans le groupe comprenant les radicaux alcoxy en $C_1$ à $C_5$, un groupe $NO_2$ et halogène;

m étant égal à 0 ou à un nombre entier de 1 à 3.

**2.** Utilisation selon la revendication 1, dans laquelle $R_1$ et $R_2$ sont indépendamment choisis parmi les radicaux linéaires ou ramifiés alkyle en $C_1$ à $C_{10}$, aryle en $C_6$ à $C_{14}$, arylalkyle en $C_7$ à $C_{18}$ ou alkylaryle en $C_7$ à $C_{18}$, ou encore $R_1$ et $R_2$ pris ensemble forment un cycle cycloalkyle en $C_3$ à $C_{10}$, avec l'atome de carbone auquel ils sont liés, et/ou $R_3$ consiste en un radical alkyle en $C_1$ à $C_5$ ou alcoxy en $C_1$ à $C_5$, et R' est choisi dans le groupe comprenant les radicaux alkyle en $C_1$ à $C_{20}$, cycloalkyle en $C_3$ à $C_{12}$, aryle en $C_6$ à $C_{14}$, alkylaryle en $C_7$ à $C_{20}$, arylalkyle en $C_7$ à $C_{20}$ et les radicaux hétérocycliques contenant 5 ou 6 atomes et R' pouvant être éventuellement substitué par au moins un groupe choisi parmi les radicaux alcoxy en $C_1$ à $C_5$, un groupe $NO_2$ et halogène.

**3.** Utilisation selon l'une quelconque des revendications précédentes, dans laquelle $R_3$ est R'CO.

**4.** Utilisation selon la revendication 1, dans laquelle les composés oxaziridine consistent en:
- 2-benzoyl-3,3-pentaméthylène-oxaziridine;
- 2-benzoyl-3-méthyl-3-éthyl-oxaziridine;
- 2-[(4-benzoyl)-benzoyl]-3,3-pentaméthylène-oxaziridine;
- 2-[(4-benzoyl)-benzoyl]-3-méthyl-3-éthyl-oxaziridine;
- 2-[(4-méthyl)-benzoyl]-3-méthyl-3-éthyl-oxaziridine;
- 2-[(4-nitro)-benzoyl]-3-méthyl-3-propyl-oxaziridine;
- 2-benzoyl-3-méthyl-3-isopropyl-oxaziridine;
- 2-[(4-chloro)-benzoyl]-3-méthyl-3-éthyl-oxaziridine;

ainsi que leurs mélanges.

**5.** Composition photopolymérisable comprenant au moins un substrat polymérisable à saturation éthylénique et au moins un composé oxaziridine tel que défini dans l'une quelconque des revendications 1 à 4.

**6.** Composition selon la revendication 5, dans laquelle le rapport molaire composé oxaziridine/substrat polymérisable est compris entre 1/100 et 1/2000, notamment de 1/200 à 1/1000.

**7.** Composition selon l'une quelconque des revendications 5 et 6, caractérisée en ce que le substrat à insaturation éthylénique comprend du methacrylate de méthyle et/ou du styrène.

**8.** Procédé de polymérisation d'une composition photo-polymérisable telle que définie dans l'une quelconque des revendications 5 à 7, comprenant l'exposition de ladite composition à des radiations dont la longueur d'onde est comprise entre 200 à 700 nm, de préférence à une température comprise entre 10 et 50°C, tout particulièrement comprise entre 20 et 30°C, pendant 30 secondes à 20 heures.

**9.** Polymères pouvant être obtenus par photopolymérisa-tion des compositions selon l'une quelconque des revendications 5 à 7.